(19) Europäisches Patentamt / European Patent Office / Office européen des brevets

(11) EP 3 617 225 A1

(12) **EUROPEAN PATENT APPLICATION**
published in accordance with Art. 153(4) EPC

(43) Date of publication:
04.03.2020 Bulletin 2020/10

(21) Application number: 17906822.6

(22) Date of filing: 01.08.2017

(51) Int Cl.:
C07K 14/47 (2006.01)   C12N 15/62 (2006.01)
C12N 15/70 (2006.01)   A61K 8/64 (2006.01)
A61Q 19/08 (2006.01)

(86) International application number:
PCT/KR2017/008298

(87) International publication number:
WO 2018/199393 (01.11.2018 Gazette 2018/44)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 26.04.2017 KR 20170053909

(71) Applicants:
• Nexgen Biotechnologies, Inc.
  Seoul 08376 (KR)
• Lee, Sun Kyo
  Gyeonggi-do 14241 (KR)

(72) Inventors:
• LEE, Sun Kyo
  Gwangmyeong-si
  Gyeonggi-do 14241 (KR)
• LEE, Seong Ran
  Gwangmyeong-si
  Gyeonggi-do 14242 (KR)
• RYU, Han Bong
  Seoul 07019 (KR)
• KIM, Tae Hyun
  Bucheon-si
  Gyeonggi-do 14685 (KR)
• CHOI, Tae Won
  Seoul 08232 (KR)

(74) Representative: Nony
11 rue Saint-Georges
75009 Paris (FR)

(54) **FUSION PROTEIN OF HEAT SHOCK PROTEIN HAVING INCREASED ANTIOXIDANT ACTIVITY AND SKIN CELL PROLIFERATION EFFECT AND COSMETIC COMPOSITION CONTAINING SAME AS ACTIVE INGREDIENT FOR IMPROVING SKIN WRINKLES**

(57) The present invention relates to a fusion protein of heat shock proteins which has an increased anti-oxidant activity and skin cell proliferation effect and an anti-wrinkle composition which comprises the fusion protein as an effective component, and the cosmetic composition according to the present invention can be advantageously used in future as a material of a functional cosmetic product.

Fig. 3

**Description**

TECHNICAL FIELD

The present invention relates to a fusion protein of heat shock proteins with increased anti-oxidant activity and skin cell proliferation effect, and an anti-wrinkle cosmetic composition comprising the same as an effective component.

BACKGROUND ART

Heat shock protein is one of the proteins that are expressed in cells in response to exposure to an extreme environment to prevent damages occurring in cell. Most of heat shock proteins have a chaperon function for preventing functional loss of a protein that is caused by exposure to an extreme environment, and a great amount of energy (i.e., ATP) is required for the process. In skin, high temperature and ultraviolet ray are representative examples of the extreme environment, and, in particular, ultraviolet ray is the direct cause of skin aging. In recent years, studies are made on a heat shock protein which exhibits an ultraviolet ray-shielding effect, and various attempts are also made to use a heat shock protein as a component of a cosmetic composition.

In the present invention, a fusion protein having an excellent anti-oxidant effect and skin regeneration effect is developed according to fusion between heat shot protein 10 and heat shot protein 20, and an anti-wrinkle cosmetic composition comprising the fusion protein as an effective component is developed.

Meanwhile, in Korean Patent Application Publication No. 2016-0084825, "Composition for preventing and alleviating skin hypersensitivity caused by exposure to ultraviolet ray comprising as an effective component an extract of onion skin for suppressing expression of Hsp70, and cosmetics using the same" is disclosed, and in Korean Patent Application Publication No. 2011-0136372, "Anti-inflammatory skin composition comprising Siegesbeckia glabrescens Makino and cell-penetrating heat shock protein 27 fusion protein" is disclosed. However, the fusion protein of heat shock proteins with increased anti-oxidant activity and skin cell proliferation effect of the present invention, and an anti-wrinkle cosmetic composition comprising the fusion protein as an effective component are not disclosed before.

DETAILED DESCRIPTION OF THE INVENTION

TECHNICAL PROBLEMS TO BE SOLVED

The present invention is devised under the circumstances described above, and the inventors of the present invention prepared a novel fusion protein according to fusion between a gene encoding heat shock protein 10 and a gene encoding heat shock protein 20, in which the genes are *Escherichia coli* (*E.coli*) codon-optimized. It was found that the fusion protein prepared by the fusion between heat shock protein 10 and heat shock protein 20 has an excellent anti-oxidant effect, and, as a result of treating skin cell line with the fusion protein, a more excellent cell proliferation effect is obtained from a treatment with the fusion protein compared to a treatment with the individual proteins, and the present invention is completed accordingly.

TECHNICAL MEANS FOR SOLVING THE PROBLEMS

To solve the problems described in the above, the present invention provides a fusion protein of heat shock protein 10 and heat shock protein 20 with increased anti-oxidant activity and skin cell proliferation effect in which the fusion protein consists of the amino acid sequence of SEQ ID NO: 2 or SEQ ID NO: 4.

The present invention further provides a gene encoding the aforementioned fusion protein.

The present invention further provides a recombinant vector comprising the aforementioned gene.

The present invention further provides a host cell transformed with the aforementioned recombinant vector.

The present invention further provides a method for producing in a host cell a fusion protein of heat shock protein 10 and heat shock protein 20 including transforming a host cell with the aforementioned recombinant vector.

The present invention further provides a fusion protein of heat shock protein 10 and heat shock protein 20 produced by the aforementioned method.

The present invention still further provides an anti-wrinkle cosmetic composition comprising, as an effective component, a fusion protein of heat shock protein 10 and heat shock protein 20, which consists of the amino acid sequence of SEQ ID NO: 2 or SEQ ID NO: 4.

ADVANTAGEOUS EFFECT OF THE INVENTION

The production method of the present invention in which the production in *E.coli* is made by using a gene encoding heat shock protein 10 and a gene encoding heat shock protein 20, in which the genes are *E.coli* codon-optimized, enables a simplified production process because the recombinant fusion protein is expressed in form of an inclusion body in *E.coli*, and it also enables production of the recombinant protein in large amount. Furthermore, the fusion protein of heat shock proteins which is produced by the aforementioned method has an excellent anti-oxidant activity and an excellent skin regeneration effect, and thus it is expected that the fusion protein can be advantageously used as a raw material of a novel functional cosmetic.

BRIEF DESCRIPTION OF THE DRAWINGS

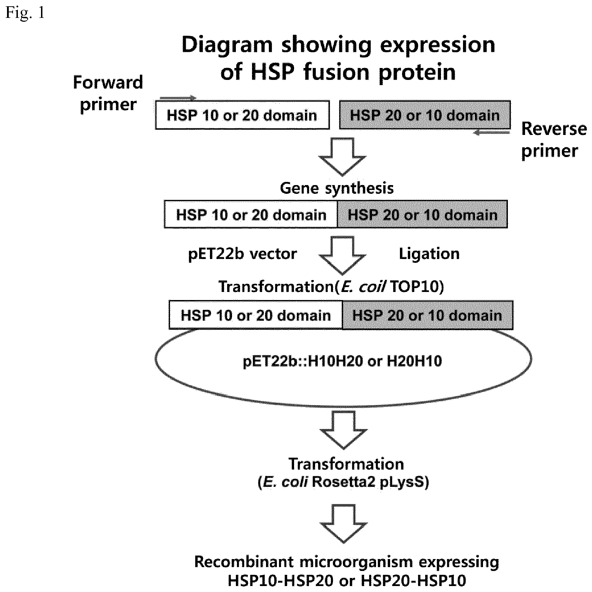

Fig. 1 is a schematic drawing illustrating the process of producing the recombinant plasmid (pET22b::H10H20 and pET22b::H20H10) which contains a gene encoding the fusion protein of heat shock protein (HSP 10 or HSP 20), and transformation of *E.coli* with the recombinant plasmid.

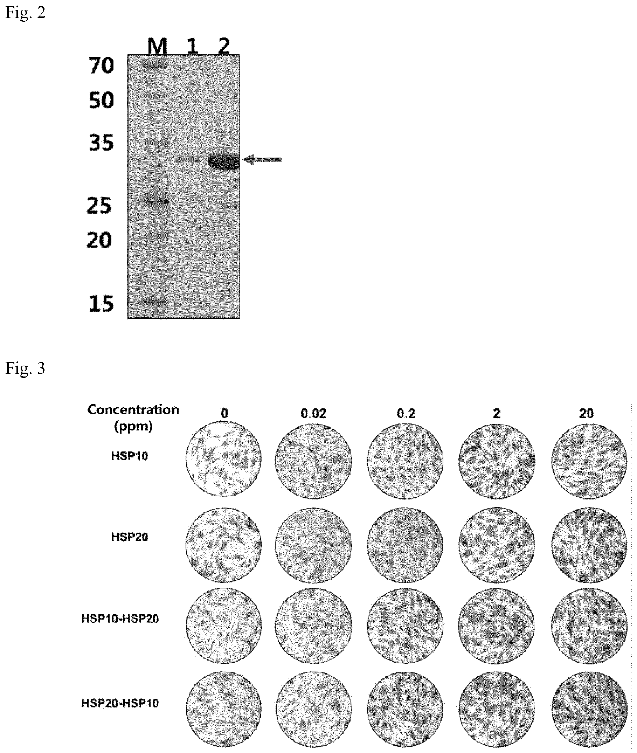

Fig. 2 is a photographic image of the SDS-PAGE gel of a fusion protein which has been finally separated and purified after expression of the fusion protein of the present invention in *E.coli*, in which M represents a size marker; 1 represents a fusion protein of heat shock protein 10 and heat shock protein 20 (HSP10-HSP20); and 2 represents a fusion protein of heat shock protein 20 and heat shock protein 10 (HSP20-HSP10).

Fig. 3 shows the result of determining the cell proliferation of dermal fibroblast cells after treating the cells only with heat shock proteins (HSP10 and HSP20) that are used for the production of a fusion protein, or with a fusion protein of heat shock proteins (HSP10-HSP20 and HSP20-HSP10), in which the determination was made based on crystal violet staining after the treatment.

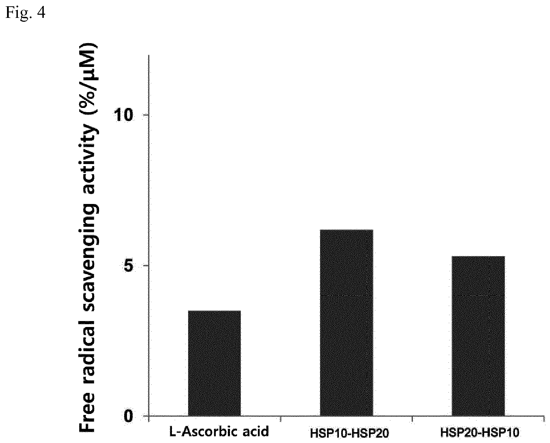

Fig. 4 shows the result of determining the anti-oxidant effect of the fusion protein of heat shock proteins.

BEST MODE(S) FOR CARRYING OUT THE INVENTION

To achieve the aforementioned object of the present invention, the present invention provides a fusion protein of heat shock protein 10 and heat shock protein 20 with increased anti-oxidant activity and skin cell proliferation effect in which the fusion protein consists of the amino acid sequence of SEQ ID NO: 2 or SEQ ID NO: 4.

A protein having the amino acid sequence represented by SEQ ID NO: 2 (i.e., heat shock protein 20 is fused to the carboxy terminal of heat shock protein 10; HSP10-HSP20) or SEQ ID NO: 4 (i.e., heat shock protein 20 is fused to the amino terminal of heat shock protein 10; HSP20-HSP10), and also functional equivalents of the protein fall within the scope of the fusion protein of heat shock protein 10 and heat shock protein 20 of the present invention. The term "functional equivalents" indicates a protein having, as a result of addition, substitution, or deletion of an amino acid, at least 70%, preferably at least 80%, more preferably at least 90%, and even more preferably at least 95% sequence homology with the amino acid sequence represented by SEQ ID NO: 2 or SEQ ID NO: 4, and it indicates a protein exhibiting substantially the same activity as the protein represented by SEQ ID NO: 2 or SEQ ID NO: 4. The expression "substantially the same activity" means an anti-oxidant effect and a cell regeneration effect. Also included in the present invention are fragments, derivatives, and analogues of the fusion protein of heat shock protein 10 and heat shock protein 20. The terms "fragments", "derivatives", and "analogues" that are described in the present specification indicate a polypeptide with the substantially same biological function or activity as the fusion protein of heat shock protein 10 and heat shock protein 20 of the present invention.

The fusion protein of heat shock proteins of the present invention preferably consists of the amino acid sequence of SEQ ID NO: 2 or SEQ ID NO: 4, in which the fusion protein consisting of the amino acid sequence of SEQ ID NO: 2 is a novel protein that is produced by fusion between the heat shock protein 10 consisting of the 2nd to the 102nd amino acids and heat shock protein 20 consisting of the 103rd to the 283rd amino acids of SEQ ID NO: 2, while the fusion protein consisting of the amino acid sequence of SEQ ID NO: 4 is a novel protein that is produced by fusion between heat shock protein 20 consisting of the 2nd to the 182nd amino acids and heat shock protein 10 consisting of the 183rd to the 283rd amino acids of SEQ ID NO: 4.

The present invention further provides a gene encoding the fusion protein of heat shock protein 10 and heat shock protein 20 which has an increased anti-oxidant activity and skin cell regeneration effect. The gene may consist of the *E.coli* codon-optimized nucleotide sequence of SEQ ID NO: 1 or SEQ ID NO: 3, but it is not limited thereto.

This gene encoding the fusion protein of heat shock protein 10 and heat shock protein 20 with increased anti-oxidant activity and skin cell regeneration effect of the present invention may include the nucleotide sequence of SEQ ID NO: 1 (i.e., gene encoding the protein HSP10-HSP20, which is a protein in which heat shock protein 20 is fused to the carboxy terminal of heat shock protein 10) or SEQ ID NO: 3 (i.e., gene encoding the protein HSP20-HSP10, which is a protein in which heat shock protein 20 is fused to the amino terminal of heat shock protein 10). Furthermore, homologues of the nucleotide sequence are also within the scope of the present invention. Specifically, the above described gene may comprise a nucleotide sequence which has preferably at least 70%, more preferably at least 80%, still more preferably at least 90%, and most preferably at least 95% homology with the nucleotide sequence selected from a group consisting of SEQ ID NO: 1 and SEQ ID NO: 3. The "sequence homology %" for a certain polynucleotide is identified by comparing a comparative region with two sequences that are optimally aligned. In this regard, a part of the polynucleotide in comparative region may comprise an addition or a deletion (i.e., a gap) compared to a reference sequence (without any addition or deletion) relative to the optimized alignment of the two sequences.

"Codon-optimized" means a modification of codon of a polynucleotide encoding a protein with a codon that is used first than others in a specific organism such that the coded protein can be more efficiently expressed therein. Because most amino acids are described by several codons that are referred to as "synonym" or "synonymous codon", genetic codes have degeneracy. However, codon usage by a specific organism is not random, and it is rather biased to specific codon triplets. Such codon usage bias may be even higher in relation with a certain gene, a gene with common function or ancestor origin, protein expressed at high level vs. proteins with low copy number, or a group protein coding region of a genome of an organism. The nucleotide sequence of SEQ ID NO: 1 or SEQ ID NO: 3 of the present invention is a sequence which has been optimized to *E.coli* codon such that the gene encoding the fusion protein of heat shock protein 10 and heat shock protein 20 can be expressed in *E. coli*.

The present invention further provides a recombinant vector comprising the aforementioned gene, and a host cell transformed with the recombinant vector.

The term "recombinant" indicates a cell which replicates a heterogeneous nucleotide or expresses said nucleotide, or a peptide, a heterogeneous peptide, or a protein encoded by a heterogeneous nucleotide. Recombinant cell can express a gene or a gene fragment in the form of a sense or antisense, which are not found in natural state of cell. In addition, a recombinant cell can express a gene that is found in natural state, provided that said gene is modified and re-introduced into the cell by an artificial means.

According to the present invention, the gene encoding a fusion protein of heat shock protein 10 and heat shock protein 20 can be inserted to a recombinant expression vector. The term "recombinant expression vector" means bacteria plasmid, phage, yeast plasmid, plant cell virus, mammalian cell virus, or other vector. In general, any plasmid and vector can be used if it can replicate and be stabilized in a host. Important characteristics of the expression vector include that it comprises a replication origin, a promoter, a marker gene, and a translation control element.

The expression vector comprising the gene sequence encoding a fusion protein of heat shock protein 10 and heat shock protein 20 and an appropriate signal for regulating transcription/translation can be constructed according to a method which is well known to a skilled person in the art. The method includes an *in vitro* recombinant DNA technique, a DNA synthesis technique, and an *in vivo* recombinant technique. For inducing mRNA synthesis, the DNA sequence can be effectively linked to a suitable promoter present in the expression vector. In addition, the expression vector may comprise a ribosome binding site as a translation initiation site and a transcription terminator.

The recombinant vector according to one embodiment of the present invention is prepared by in-frame fusion of 5' terminal (*Nde*I restriction enzyme site) and 3' terminal (*Xho*I restriction enzyme site) of a synthesized gene encoding the fusion protein of heat shock proteins (i.e., SEQ ID NO: 1 or SEQ ID NO: 3) to pET22b vector, and it may be a recombinant vector characterized in that it can produce the fusion protein of heat shock proteins based on effective expression of the aforementioned gene with an aid of lac promoter (*lac* promoter) and lacI repressor (*lac*I repressor), but it is not limited thereto.

For a host cell having an ability of having stable and continuous cloning and expression of the vector of the present invention in a prokaryotic cell, any host cell known in the pertinent art can be used. Examples of the prokaryotic cells include, Bacillus sp. strain including *E.coli* Rosetta, *E.coli* JM109, *E.coli* BL21, *E.coli* RR1, *E.coli* LE392, *E.coli* B, *E.coli* X 1776, *E.coli* W3110, *Bacillus subtillus, Bacillus thuringiensis* and the like, and intestinal bacteria and strains including *Salmonella typhimurium, Serratia marcescens* and various *Pseudomonas sp.* etc.

Furthermore, when an eukaryotic cell is transformed with the vector of the present invention, yeast (*Saccharomyce cerevisiae*), an insect cell, a human cell (for example, CHO (Chinese hamster ovary) cell line, W138, BHK, COS-7, HEK 293, HepG2, 3T3, RIN, and MDCK cell line), a plant cell, and the like can be used as a host cell.

The transformed host cell of the present invention may be *E.coli* Rosetta2 (DE3) pLysS cell line, but it is not limited thereto.

When a host cell is a prokaryotic cell, delivery of the vector of the present invention into a host cell can be carried out by $CaCl_2$ method, Hanahan's method (Hanahan, D., J. Mol. Biol., 166:557-580 (1983)) or an electroporation method, and the like. In addition, when a host cell is an eukaryotic cell, the vector can be introduced to a host cell by a microinjection method, calcium phosphate precipitation method, an electroporation method, a liposome-mediated transfection method, DEAE-dextran treatment method, or a gene bombardment method, and the like.

The present invention further provides a method for producing in a host cell a fusion protein of heat shock protein 10 and heat shock protein 20 including transforming a host cell with the aforementioned recombinant vector to overexpress the gene encoding a fusion protein of heat shock protein 10 and heat shock protein 20, and it further provides a fusion protein of heat shock protein 10 and heat shock protein 20 that is produced by the aforementioned method.

In the method according to one embodiment of the present invention, the host cell may be preferably *E. coli,* and more preferably *E.coli* Rosetta2 (DE3) pLysS cell line, but it is not limited thereto.

The present invention still further provides an anti-wrinkle cosmetic composition comprising, as an effective component, a fusion protein of heat shock proteins which consists of the amino acid sequence of SEQ ID NO: 2 or SEQ ID NO: 4.

In the cosmetic composition according to one embodiment of the present invention, content of the fusion protein of heat shock protein 10 and heat shock protein 20 may be 0.000001 to 0.00002% by weight relative to the total weight of the cosmetic composition, but it is not limited thereto.

In the cosmetic composition of the present invention, components that are typically used for a cosmetic composition are included in addition to the effective component described above. Examples thereof include a lipid material, an organic solvent, a dissolution agent, a condensation agent, a gelling agent, a softening agent, an anti-oxidant, a suspension agent, a stabilizer, a foaming agent, an aroma, a surface active agent, water, an ionic or non-ionic emulsifier, a filler, a metal ion sequestering agent, a chelating agent, a preservative, vitamin, a blocking agent, a moisturizing agent, essential oil, a dye, a pigment, a hydrophilic or liphophilic activating agent, a common auxiliary agent such as lipid vesicle, and a carrier.

The composition of the present invention can be prepared in any formulation which is generally prepared in the pertinent art. For example, the composition may be formulated into a solution, a suspension, an emulsion, a paste, a gel, a crème, a lotion, a powder, an oil, a powder foundation, an emulsion foundation, a wax foundation, a spray, or the like, but not limited thereto. More specifically, the composition may be formulated into a skin, a skin softener, a skin toner, an astringent, a lotion, a milk lotion, a moisture lotion, a nutrition lotion, a massage crème, a nutrition crème, an eye crème, a moisture crème, a hand crème, an essence, a nutrition essence, a pack, a cleansing foam, a cleansing water, a cleansing lotion, a cleansing crème, a body lotion, a body cleanser, a soap, a powder, or the like.

In a case in which the cosmetic composition of the present invention has a formulation type of paste, crème, or gel, it is possible to use, as a carrier component, animal oil, plant oil, wax, paraffin, starch, tragacanth, cellulose derivatives, polyethylene glycol, silicone, bentonite, silica, talc, or zinc oxide.

In a case in which the cosmetic composition of the present invention has a formulation type of powder or spray, it is possible to use, as a carrier component, lactose, talc, silica, aluminum hydroxide, calcium silicate, or polyamide powder, when it is spray, in particular, a propellant such as chlorofluoro hydrocarbon, propane/butane, or dimethyl ether may be additionally contained.

In a case in which the cosmetic composition of the present invention has a formulation type of solution or emulsion, a solvent, a dissolution agent, or an emulsifier is used as a carrier component, and examples thereof include water, ethanol, isopropyl alcohol, ethyl carbonate, ethyl acetate, benzyl alcohol, benzyl benzoate, propylene glycol, 1,3-butyl glycol oil, glycerol aliphatic ester, polyethylene glycol, and fatty acid ester of sorbitan.

In a case in which the cosmetic composition of the present invention has a formulation type of suspension, it is possible to use, as a carrier component, a liquid phase diluent such as water, ethanol, or propylene glycol, a suspension agent such as ethoxylated isostearyl alcohol, polyoxyethlyene sorbitol ester, or polyoxyethylene sorbitan ester, microcrystalline cellulose, aluminum metahydroxide, bentonite, agar, or tragacanth.

Hereinbelow, the present invention is explained in greater detail in view of the Examples. However, it is evident that the following Examples are given only for exemplification of the present invention and by no means the present invention is limited to the following Examples.


EXAMPLES


Example 1. Preparation of recombinant expression vector and transformed recombinant microorganism for producing fusion protein of heat shock proteins


The optimized gene encoding the fusion protein of heat shock protein 10 and heat shock protein 20, recombinant expression vector, and transformed recombinant microorganism were produced according to the following methods.

By using as a template a gene encoding heat shock protein 10 or heat shock protein 20, a gene (SEQ ID NO: 1 or SEQ ID NO: 3) encoding a fusion protein of heat shock protein 10 and heat shock protein 20 (SEQ ID NO: 2 or SEQ ID NO: 4), which consists of 238 amino acids and is optimized such that it can be expressed in a host microorganism, was synthetically prepared. The primers used for the preparation were as follows.

The primers for synthesizing a gene encoding the HSP10-HSP20 fusion protein in which heat shock protein 20 is fused to the carboxy terminal of heat shock protein 10 are described below.

Forward primer: 5'-CATATGGCGGGTCAGGCG-3' (SEQ ID NO: 5)
Reverse primer: 5'-CTCGAGCGGTTCAAC-3' (SEQ ID NO: 6)
Sense primer: 5'-TACGTTGACTCTGGTCGTTC-3' (SEQ ID NO: 7)

Anti-sense primer: 5'-GAACGACCAGAGTCAACGTA-3' (SEQ ID NO: 8)

The primers for synthesizing a gene encoding the HSP20-HSP10 fusion protein in which heat shock protein 20 is fused to the amino terminal of heat shock protein 10 are described below.

Forward primer: 5'-CATATGTCTGGTCGTTCT-3' (SEQ ID NO: 9)
Reverse primer: 5'-CTCGAGGTCAACGTA-3' (SEQ ID NO: 10)
Sense primer: 5'-GTTGAACCGGCGGGTCAG-3' (SEQ ID NO: 11)
Anti-sense primer: 5'-CTGACCCGCCGGTTCAAC-3' (SEQ ID NO: 12)

According to digestion of the above gene fragment and recombinant plasmid with the same restriction enzymes (5' terminus *Nde*I and 3' terminus *Xho*I) followed by insertion, the recombinant plasmid (pET22b::H10H20 and pET22b::H20H10) shown in Fig. 1 was prepared. By transforming *E.coli* TOP10 with the prepared recombinant plasmid, the gene construct was obtained in large amount from the host organism.

After that, *E.coli* Rosetta2 (DE3) pLysS (Novagen, Germany) was transformed with the prepared recombinant plasmid so that a recombinant microorganism for producing the fusion protein of heat shock protein 10 and heat shock protein 20 was prepared.

Example 2. Induced expression, separation, and purification of fusion protein of heat shock proteins

*E.coli* Rosetta2 (DE3) pLysS prepared in Example 1 was cultured by using 1 $\ell$ LB medium (10% tryptophan, 10% sodium chloride, and 5% yeast extract) or BSS medium (1% tryptophan, 0.5% yeast extract, 1% glucose, and 0.1% HEPES (pH 7.0), Nexgen Biotechnologies, Inc.) till to have $OD_{600}$=0.6 to 0.8 for batch culture, or $OD_{600}$=15 to 20 for continuous culture which uses a 20 $\ell$ fermenter. After that, by adding 1 to 5 mM IPTG or 2% lactose (each at final concentration) to the cell culture medium, gene expression of the recombinant *E.coli* was induced. After inducing the gene expression, the cells were additionally cultured for 3 to 4 hours, and then collected by centrifuge. The resulting cells were completely suspended in phosphate buffered saline (8 g sodium chloride, 0.2 g potassium chloride, 1.44 g sodium hydrogen phosphate ($Na_2HPO_4$), and 0.24 g potassium dihydrogen phosphate ($KH_2PO_4$)/$\ell$, pH 7.4), and then disrupted by using an ultrasonic homogenizer so as to separate a solution containing the intracellular proteins. By using thus-separated solutions as a sample, protein expression was determined by 15% SDS-polyacrylamide gel electrophoresis. As a result, it was found that the fusion protein of heat shock protein 10 and heat shock protein 20 is expressed in a crude lysate of cells which have been induced to undergo the expression by IPTG or lactose.

In order to separate and purify the fusion protein of heat shock protein 10 and heat shock protein 20 of which expression has been confirmed, the inclusion body was solubilized with a solubilizing buffer solution (5 M urea, pH 11), and then subjected to a refolding process by ultrafine filtration (0.45 $\mu$m fine filtration membrane and 1 K ultrafine filtration membrane). By using a buffer solution for storage (PBS), the fusion protein of heat shock protein 10 and heat shock protein 20 was finally separated.

For having complete purification of the above fusion protein of heat shock proteins, the separated fusion protein was passed through a nickel-agarose column at a rate of 1 to 3 ml/minute. Subsequently, the column was washed several times with a binding buffer solution, and an imidazole solution (pH 7.4) at a concentration of 50, 100, or 250 mM was applied to the column to fractionate and elute the fusion protein of heat shock protein 10 and heat shock protein 20, in which each fraction is eluted in an amount of 1 ml. Then, the imidazole in the buffer was removed by using 10 mM potassium phosphate solution so that the fusion protein was finally purified in pure state. To examine the result, 15% SDS-polyacrylamide gel electrophoresis was carried out. As a result, the finally purified fusion protein was found near the region having the expected size (about 30 kDa including His tag) (Fig. 2).

Example 3. Activity measurement of fusion protein of heat shock proteins: Dermal fibroblast cell proliferation effect

After selecting the samples from which the presence of the fusion protein of heat shock protein 10 and heat shock protein 20 has been confirmed as the fusion protein is separated and purified in Example 2, activity of the fusion protein was measured.

Dermal fibroblast cells (Human Dermal Fibroblasts adult, HDFa cell) were cultured, and then treated with the individual heat shock protein (HSP10, HSP20), which has been used for the preparation of a fusion protein, or with the fusion protein of heat shock protein 10 and heat shock protein 20 (HSP10-HSP20, HSP20-HSP10), each at a concentration of 0, 0.02, 0.2, 2.0, or 20 ppm, followed by culture for 3 days at 37°C. After that, proliferation of the dermal fibroblast cells was examined by crystal violet staining.

As a result, it was found that a more excellent dermal fibroblast proliferation effect is obtained as the concentration of the fusion protein of heat shock proteins increases (Fig. 3). It was also observed that, compared to the group treated with a single protein (i.e., HSP10 or HSP20), the fusion protein of heat shock protein 10 and heat shock protein 20 (HSP10-HSP20, HSP20-HSP10) exhibits a higher cell proliferation effect. Because the number of amino acids is 283 for the fusion protein while the number of amino acid is 101 or 181 for heat shock protein 10 and heat shock protein 20, respectively, the mole number of the fusion protein is about 1/3 to 2/3 of the each single protein of heat shock protein 10 and heat shock protein 20 when the treatment is carried out with a single protein or the fusion protein, which are present at the same concentration (for example, 0.02 ppm). Accordingly, it is recognized that, if a similar dermal fibroblast proliferation effect is exhibited at the same concentration, the fusion protein has a dermal fibroblast proliferation effect that is 1.5 to 3 times higher than the single protein (HSP10). As it can be realized from Fig. 3, compared to the treatment with a single protein, the dermal fibroblast proliferation effect is higher when the treatment is carried out with the fusion protein of heat shock protein 10 and heat shock protein 20, and thus the fusion protein is found to have a dermal fibroblast proliferation effect that is at least 1.5 to 3 times higher than the single protein. Based on this result, it is recognized that the fusion protein of heat shock protein 10 and heat shock protein 20 of the present invention has an excellent dermal cell proliferation effect.

Example 4. Activity measurement of fusion protein of heat shock proteins: Anti-oxidant effect

In order to examine the anti-oxidant effect of the fusion protein of heat shock protein 10 and heat shock protein 20, DPPH (1,1-diphenyl-2-pycryl-hydrazyl) method, which is one of the methods for measuring the free radical scavenging activity, was employed.

In order to examine the anti-oxidant activity of the fusion protein of heat shock protein 10 and heat shock protein 20, L-ascorbic acid was used as a control group. For the test, a fusion protein of heat shock proteins and L-ascorbic acid were prepared each at 1 μM concentration while DPPH was prepared at concentration of 0.2 mM. After mixing each of them at a ratio of 1 : 1, they were allowed to stand for 30 minutes at 37°C. After that, the absorbance at 520 nm was measured by using an ELISA reader. The free radical scavenging activity (%) was calculated based on the following equation 1, and the results are shown in Fig. 4.

[Equation 1]

$$\text{Free radical scavenging activity (\%)} = 100-((B/A)*100)$$

A: Absorbance by control group which has not been treated with any test sample
B: Absorbance by test group which has been treated with test sample

As a result, it was shown that the fusion protein of heat shock proteins showed the free radical scavenging activity which is higher by approximately 1.5 to 1.8 times than L-ascorbic acid as a positive control group. Thus, it is found that the fusion protein of heat shock proteins of the present invention has a very high anti-oxidant activity, and it indicates that the fusion protein has a skin aging preventing effect based on the anti-oxidant activity.

**Claims**

1. A fusion protein of heat shock protein 10 and heat shock protein 20 with increased anti-oxidant activity and skin cell proliferation effect in which the fusion protein consists of the amino acid sequence of SEQ ID NO: 2 or SEQ ID NO: 4.

2. A gene encoding the fusion protein of heat shock protein 10 and heat shock protein 20 of Claim 1 with increased anti-oxidant activity and skin cell proliferation effect.

3. The gene according to Claim 2, wherein the gene consists of the nucleotide sequence of SEQ ID NO: 1 or SEQ ID NO: 3 that is *E.coli* codon-optimized.

4. A recombinant vector comprising the gene of Claim 2 or 3.

5. A host cell transformed with the recombinant vector of Claim 4.

6. A method for producing in a host cell a fusion protein of heat shock protein 10 and heat shock protein 20 including transforming a host cell with the recombinant vector of Claim 4 to overexpress a gene encoding a fusion protein of heat shock protein 10 and heat shock protein 20.

7. The method for producing a fusion protein of heat shock protein 10 and heat shock protein 20 according to Claim 6, wherein the host cell is *E. coli.*

8. An anti-wrinkle cosmetic composition comprising, as an effective component, a fusion protein of heat shock protein 10 and heat shock protein 20 with increased anti-oxidant activity and skin cell proliferation effect which consists of the amino acid sequence of SEQ ID NO: 2 or SEQ ID NO: 4.

Fig. 1

# Diagram showing expression
# of HSP fusion protein

Forward primer

| HSP 10 or 20 domain | HSP 20 or 10 domain |

Reverse primer

Gene synthesis

| HSP 10 or 20 domain | HSP 20 or 10 domain |

pET22b vector  Ligation

Transformation(*E. coil* TOP10)

| HSP 10 or 20 domain | HSP 20 or 10 domain |

pET22b::H10H20 or H20H10

Transformation
(*E. coli* Rosetta2 pLysS)

Recombinant microorganism expressing
HSP10-HSP20 or HSP20-HSP10

Fig. 2

Fig. 3

Fig. 4

## INTERNATIONAL SEARCH REPORT

International application No.

PCT/KR2017/008298

| A. | CLASSIFICATION OF SUBJECT MATTER |
|---|---|

*C07K 14/47(2006.01)i, C12N 15/62(2006.01)i, C12N 15/70(2006.01)i, A61K 8/64(2006.01)i, A61Q 19/08(2006.01)i*

According to International Patent Classification (IPC) or to both national classification and IPC

| B. | FIELDS SEARCHED |
|---|---|

Minimum documentation searched (classification system followed by classification symbols)

C07K 14/47; A61K 39/395; A61K 8/64; C07K 14/46; C07K 14/485; C07K 1/10; A61K 38/16; A61K 8/67; C12N 15/62; C12N 15/70; A61Q 19/08

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: heat shock protein 10, heat shock protein 20, fusion protein, antioxidant activity, skin cell proliferation

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | US 2001-0005713 A1 (YOUNG, R. A.) 28 June 2001<br>See claims 1-3; paragraphs [0022] and [0041]-[0051]. | 1-8 |
| Y | US 2010-0015126 A1 (GEBBINK, M. F. B. G. et al.) 21 January 2010<br>See claim 18; paragraphs [0001], [0052], [0072] and [0118]. | 1-8 |
| Y | KR 10-2005-0040595 A (LG HOUSEHOLD & HEALTH CARE LTD.) 03 May 2005<br>See claim 1. | 1-8 |
| A | KR 10-1652956 B1 (NEXGEN BIOTECHNOLOGIES, INC.) 31 August 2016<br>See the entire document. | 1-8 |
| A | KR 10-1678392 B1 (NEXGEN BIOTECHNOLOGIES, INC.) 22 November 2016<br>See the entire document. | 1-8 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 JANUARY 2018 (24.01.2018) | **25 JANUARY 2018 (25.01.2018)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea | |
| Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2017/008298**

| Patent document cited in search report | Publication date | Patent family member | Publication date |
|---|---|---|---|
| US 2001-0005713 A1 | 28/06/2001 | CA 2282426 A1<br>EP 1047451 A1<br>US 2004-0204363 A1<br>WO 98-35705 A1 | 20/08/1998<br>02/11/2000<br>14/10/2004<br>20/08/1998 |
| US 2010-0015126 A1 | 21/01/2010 | CA 2645930 A1<br>EP 2007800 A1<br>WO 2007-108675 A1 | 27/09/2007<br>31/12/2008<br>27/09/2007 |
| KR 10-2005-0040595 A | 03/05/2005 | NONE | |
| KR 10-1652956 B1 | 31/08/2016 | NONE | |
| KR 10-1678392 B1 | 22/11/2016 | NONE | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20160084825

- KR 20110136372

**Non-patent literature cited in the description**

- **HANAHAN, D.** *J. Mol. Biol.,* 1983, vol. 166, 557-580